# EUROPEAN PATENT SPECIFICATION

(11) **EP 4 588 505 B1**
(45) Date of publication and mention of the grant of the patent: **28.10.2026**
(21) Application number: 25152809.7
(22) Date of filing: 20.01.2025
(51) Int. Cl.: A61N 1/372, A61B 5/00, A61N 1/375, A61N 1/39

(54) **ACTIVE MEDICAL DEVICE WITH A DUAL BAND ANTENNA CONFIGURATION HOUSED IN A NON-CONDUCTIVE ENCLOSURE CONNECTED TO A CELL CASING**
AKTIVE MEDIZINISCHE VORRICHTUNG MIT EINER ZWEIBANDANTENNENKONFIGURATION IN EINEM MIT EINEM ZELLGEHÄUSE VERBUNDENEN NICHTLEITENDEN GEHÄUSE
DISPOSITIF MÉDICAL ACTIF À CONFIGURATION D'ANTENNE DOUBLE BANDE LOGÉE DANS UNE ENCEINTE NON CONDUCTRICE RELIÉE À UN BOÎTIER DE CELLULE

(30) Priority: 18.01.2024 US 202463622212 P
(43) Date of publication of application: 23.07.2025
(73) Proprietor: Greatbatch Ltd., Clarence, NY 14031 (US)
(72) Inventor: BARBAT, Gabriel Amilcar, 15800 Ciudad de la Costa (UY); DUARTE, Camila Maria, 11400 Montevideo (UY); CURIEL, Rocio Belen, 11300 Montevideo (UY)
(74) Representative: Mathys & Squire

(56) References cited:
- EP-A1- 4 124 358
- AU-A1- 2018 265 016
- US-A1- 2016 067 477
- US-A1- 2023 372 720

## Description

### CROSS-REFERENCE TO RELATED APPLICATION

This application claims priority to U.S. provisional application Serial No. 63/622,212, filed on January 18, 2024.

### FIELD OF THE INVENTION

The present disclosure relates to the fields of implantable and externally worn medical devices. More particularly, the disclosure invention relates to an active medical device (AMD) that may be detachably connected to a lead. The lead is preferably an implantable lead that is designed to send electrical stimulation pulses to the surrounding body tissue of a patient, sense biological signals from the body tissue, or both stimulate and sense.

The AMD of the present disclosure has a power source connected to the distal end of a printed circuit board (PCB) assembly for powering its electronic components. The electronic components control the various functions of the medical device including, but not limited to, electrical stimulation of body tissue and biological sensing. A lateral edge of the PCB is connected to a communication antenna/inductive charging coil assembly that provides for remote two-way communication with an external device and inductive wireless charging of the power source. Having the communication antenna/inductive charging coil assembly connected to a lateral edge of the PCB means that the subassembly can be folded over the PCB to provide a compact PCB assembly that fits inside a, for instance low-profile ceramic, housing. The power source connected to the distal end of the PCB assembly typically also has a low-profile. A proximal end of the housing for the PCB assembly is connected to a feedthrough connector assembly which, in turn, may be connected to a strain-relief device for a lead, preferably an implantable lead. Since the communication antenna/inductive charging coil assembly is a lateral extension of the PCB, the AMD may have a relatively thin or low-profile with a reduced total volume for less invasive therapies. Preferably, an AMD according to the present disclosure has a volume of less than 3 cc. including its power source.

### BACKGROUND OF THE INVENTION

It is typical for an AMD that is powered by a rechargeable power source to support its communication antenna/inductive charging coil assembly in an epoxy header connected to the device housing or to support the subassembly on an external surface of the housing. This means that the communication antenna/inductive charging coil assembly supported in the epoxy header is typically spaced from the PCB contained inside the device housing, which requires electrical connections from the PCB to the subassembly. In addition to the epoxy header, these electrical connections add bulk to the AMD, which can be significant. Otherwise, mounting the communication antenna/inductive charging coil assembly on an outer surface of the device housing also increases the thickness of the AMD, which is added bulk.

Moreover, when the communication antenna/inductive charging coil assembly is mounted on the device housing, the metallic material of the housing behind the assembly can cause eddy currents which can negatively affect remote inductive or RF charging signals and severely limit communication between the medical device and a remote programmer or interrogation device. Remote programmers are used by the patient and the clinician to configure the medical device to operate in a desired manner. A patient programmer is used by the patient in whom the medical device is implanted to adjust the parameters of electrical stimulation delivered by the device. A clinician programmer is used by medical personnel to configure and adjust stimulation parameters that the patient is not permitted to control. European patent application publication EP 4 124 358 A1 discloses an active implantable medical device that is a battery- or capacitor-powered device designed to deliver electrical stimulation to a patient or to sense biological signals from body tissue.

Therefore, there is a desire to minimize the total volume of an AMD so that the medical device is as small as possible. According to the present disclosure, a considerable reduction in volume is realized by connecting the communication antenna/inductive charging coil assembly to a lateral edge of the PCB and then folding the assembly over the PCB. The resulting relatively thin or low-profile PCB assembly is contained inside a ceramic housing of the medical device. A distal end of the ceramic housing is connected to the casing for a power source powering the electronic components of the PCB. A proximal end of the ceramic housing is connected to a feedthrough connector assembly which is detachably connected to a strain relief device for a lead, preferably an implantable lead. The resulting AMD is significantly smaller than an AMD having a metallic housing, which is beneficial for patient safety and comfort. Preferably, the AMD of the present disclosure has a volume of less than 3 cc. including its power source.

### SUMMARY OF THE INVENTION

The present invention is defined by the appended claims. Further aspects, embodiments and examples described hereinafter are only of exemplary nature and are presented for better understanding the present invention.

The purpose of the present disclosure is to reduce the size of an active medical device (AMD), preferably an active implantable medical device (AIMD), by reducing the volume that is occupied by the communication antenna/inductive charging coil assembly connected to a printed circuit board (PCB). Unlike the conventional constructions describes in the Prior Art section above, the communication antenna/inductive charging coil assembly, whether there are two dedicated antennas/coils or an integrated antenna, are contained inside a, typically ceramic, housing of the AMD. Alumina is a preferred ceramic as it is an inherently electrically insulative material and readily permits two-way communication and inductive charging without creating detrimental eddy currents. Since both the traditional epoxy header and the alternative method of mounting the communication antenna/inductive charging coil assembly on an outer surface of the device housing are eliminated with the present AMD, a medical device of a significantly reduced size is the result. A smaller device is easier to implant in a patient and would be expected to cause less trauma to the patient. A smaller medical device would also be expected to be less cumbersome when worn on a patient's body.

In that respect, the AMD of the present disclosure has a non-conductive housing that is generally made from a ceramic material, for example, alumina, which is an inherently electrically insulative material. The ceramic housing contains a PCB assembly hingedly connected to a PCB and a communication antenna/inductive charging coil assembly. The PCB has electronic circuits that draw power from an electrical power source connected to a distal end of the ceramic housing. A lead, preferably an implantable lead, may be detachably connected to a feedthrough connector assembly at a proximal end of the ceramic housing. In that manner, electrodes of the implantable lead are energized by the PCB assembly to either provide electrical stimulation to body tissue in which the medical device is implanted, sense biological signals from adjacent body tissue, or both.

The hingedly connected aspect of the present disclosure means that the communication antenna/inductive charging coil assembly is folded over or moved into a position that is aligned directly spaced above or directly spaced below the PCB assembly. In its folded and aligned position, the communication antenna/inductive charging coil assembly is aligned directly spaced above or directly spaced below the PCB assembly. The communication antenna/inductive charging coil assembly folded over the PCB assembly may then be fit into a low-profile ceramic housing of the AMD. Importantly, the ceramic housing does not interfere with communication/charging fields that are transmitted between external devices and the AMD when it is implanted in body tissue or worn outside the body. These include communication signals from patient and clinician programmers to the medical device, and inductive or RF (radio frequency) charging signals sent from an external charging transmitter to a charging antenna connected to the power source. This means that the communication and charging antennas can be hingedly connected to the PCB assembly and folded over the PCB assembly and then fitted inside the ceramic housing, which further reduces the AMD's volume and manufacturing complexity.

These and other aspects of the present disclosure will become increasingly more apparent to those skilled in the art by reference to the following detailed description and to the appended drawings.

### BRIEF DESCRIPTION OF THE DRAWINGS

FIG. 1 is a wire formed diagram of a generic human body showing a number of medical devices 100A to 100L according to the present disclosure that can either be implanted in a patient's body tissue or attached externally to the body.
FIG. 2 is a simplified block diagram of an exemplary medical system 10 according to various embodiments of the present disclosure.
FIG. 3 is a perspective view of an exemplary active medical device (AMD) 12 that is detachably connected to an implantable lead 36.
FIG. 4 is an exploded view of the exemplary AMD 12 shown in FIG. 3.
FIG. 5 is a plan view showing a printed circuit board assembly 34 comprising electronic circuits 38 mounted to a printed circuit board (PCB) 36 and with a communication antenna/inductive charging coil assembly 52 hingedly connected to a lateral edge 42 of the PCB 36 by the living hinges 62, 64 and 66.
FIG. 5A is a plan view showing the printed circuit board assembly 34 of FIG. 5 connected to a proximal end 44 of the printed circuit board 36 of the PCB assembly 34.
FIG. 5B is a plan view showing the printed circuit board assembly 34 of FIG. 5 connected to a distal end 46 of the printed circuit board 36 of the PCB assembly 34.
FIG. 6 is a perspective, partly broken away, view of the exemplary AMD 12 of FIG. 3 with the PCB assembly 34 removed from its ceramic housing 26 and with the communication antenna/inductive charging coil assembly 52 being folded over the PCB assembly.
FIG. 7 is an enlarged plan view of the exemplary AMD 12 shown in FIG. 3.

### DETAILED DESCRIPTION OF THE PREFERRED EMBODIMENTS

As used herein, the term "active medical device" means a medical device, whether implantable or worn externally, that is designed to deliver electrical stimulation to a patient's body tissue or sense biological signals from body tissue, or both stimulate and sense. An AIMD is an active medical device that is intended to be implanted in body tissue.

Further, as used herein, the term PCB assembly defines a printed circuit board (PCB) as a laminated sandwich structure of conductive and insulating layers, each with a pattern of traces, planes and other features (similar to wires on a flat surface) etched from one or more sheet layers of copper laminated onto or between sheet layers of a non-conductive substrate. PCBs are used to connect or wire electronic components to one another in an electronic circuit. Electrical components may be fixed to conductive pads on the outer layers, generally by soldering, which both electrically connects and mechanically fastens the components to the board. PCB assemblies also typically include vias, which are metal-lined drilled holes that enable electrical interconnections between conductive layers with more than a single side.

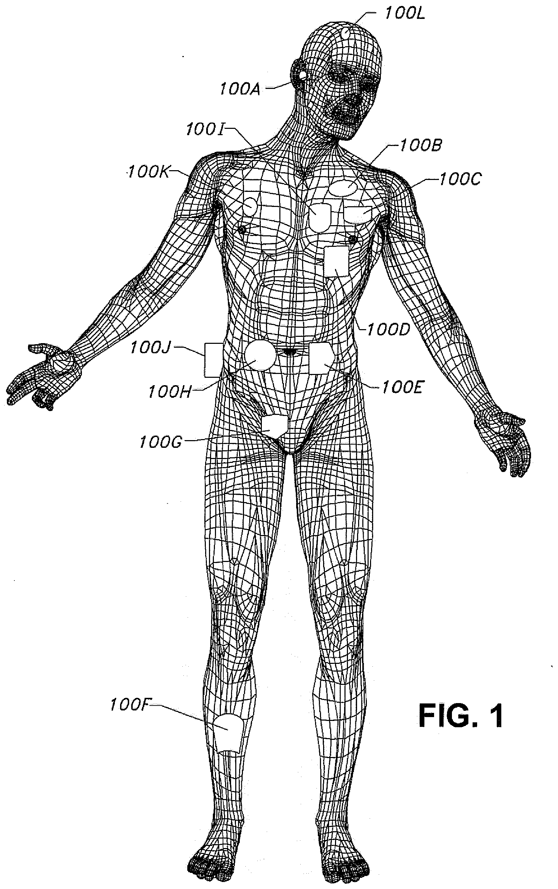

Turning now to the drawings, FIG. 1 is a wire form diagram of a generic human body illustrating various types of active implantable and external medical devices according to the present disclosure that can either be implanted in a patient's body or attached externally to the body.

Numerical designation 100A represents a family of hearing devices which can include the group of cochlear implants, piezoelectric sound bridge transducers, and the like.

Numerical designation 100B represents a variety of neurostimulators, brain stimulators, and sensors. Neurostimulators are used to stimulate the Vagus nerve, for example, to treat epilepsy, obesity, and depression. Brain stimulators are pacemaker-like devices and include electrodes implanted deep into the brain for sensing the onset of a seizure and also providing electrical stimulation to brain tissue to prevent a seizure from actually occurring. The lead wires associated with a deep brain stimulator are often placed using real time MRI imaging. Sensors include optical sensors, motion sensors, acoustic sensors, pressure sensors, analyte sensors, and electromagnetic sensors, among others.

Numerical designation 100C shows a cardiac pacemaker which is well-known in the art.

Numerical designation 100D includes the family of left ventricular assist devices (LVADs), and artificial heart devices.

Numerical designation 100E includes a family of drug pumps which can be used for dispensing insulin, chemotherapy drugs, pain medications, and the like.

Numerical designation 100F includes a variety of bone growth stimulators for rapid healing of fractures.

Numerical designation 100G includes urinary incontinence devices.

Numerical designation 100H includes the family of pain relief spinal cord stimulators and anti-tremor stimulators. Numerical designation 100H also includes an entire family of other types of neurostimulators used to block pain.

Numerical designation 100I includes a family of implantable cardioverter defibrillator (ICD) devices and also the family of congestive heart failure (CHF) devices. They are also known in the art as cardio-resynchronization therapy devices, otherwise known as CRT devices.

Numerical designation 100J illustrates an externally worn pack. This pack could be an external insulin pump, an external drug pump, an external neurostimulator or even a ventricular assist device.

Numerical designation 100K illustrates one of various types of EKG/ECG external skin electrodes which can be placed at various locations on the patient's body.

Numerical designation 100L represents external EEG electrodes that are placed on the patient's head.

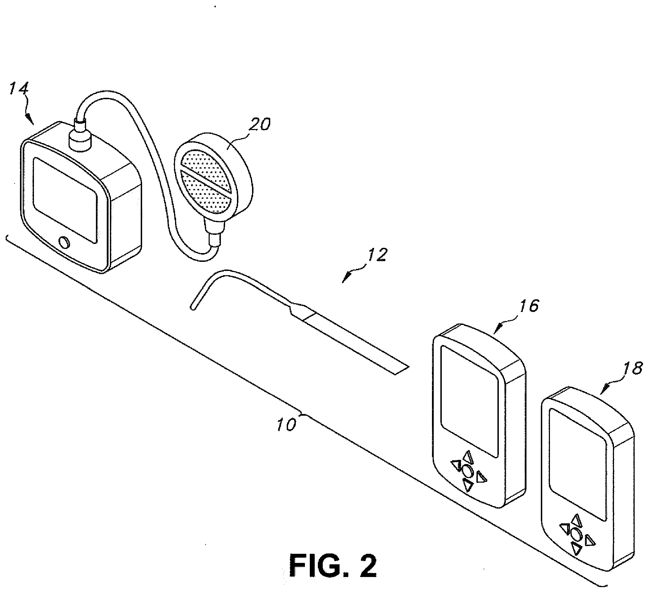

To provide context to the various medical devices 100A to 100L illustrated in FIG. 1, FIG. 2 illustrates a simplified block diagram of an exemplary medical device system 10 according to the present disclosure . The medical device system 10 includes an active medical device (AMD) 12, which is any one of various types of the medical devices that include a lead, whether implantable or external, as described above with reference to FIG. 1. The medical device system 10 also has an external charger 14, a patient programmer 16, and a clinician programmer 18.

The patient programmer 16 and the clinician programmer 18 may be portable handheld devices, such as a smartphone or other custom device, that are used to configure the AMD 12 so that the medical device can operate in a desired manner. The patient programmer 16 is used by the patient in whom the AMD 12 is implanted or worn externally. The patient may adjust the parameters of electrical stimulation delivered by the AMD 12, such as by selecting a stimulation program, changing the amplitude and frequency of the electrical stimulation, among other parameters, and by turning stimulation on and off. Additionally, the patient programmer 16 may collect and display data being collected by the medical device 12 and alert the patient to potential health risks.

The clinician programmer 18 is used by medical personnel to configure the other system components and to adjust stimulation parameters that the patient is not permitted to control. These include setting up stimulation programs among which the patient may choose and setting upper and lower limits for the patient's adjustments of amplitude, frequency, and other parameters. It is also understood that although FIG. 2 illustrates the patient programmer 16 and the clinician programmer 18 as two separate devices, they may be integrated into a single programmer in some embodiments.

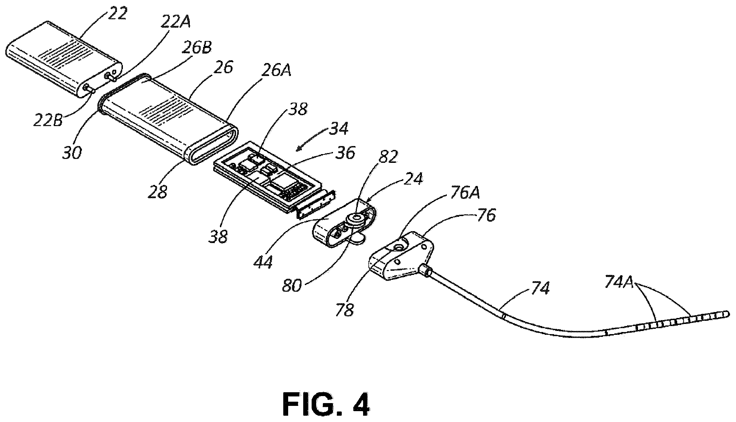

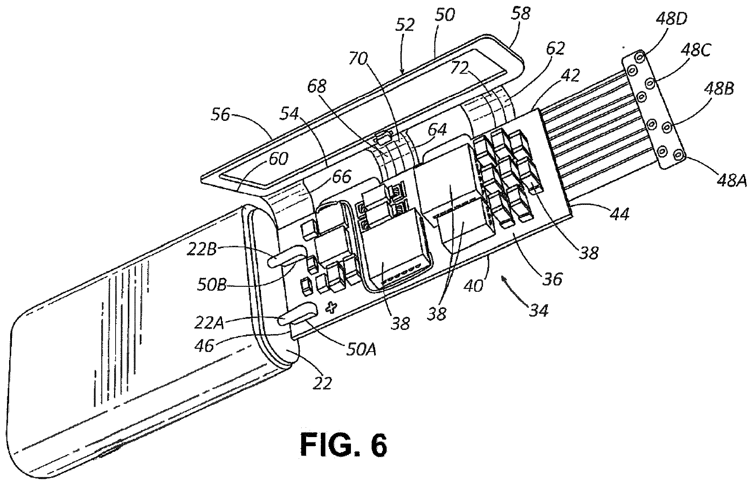

Electrical power can be delivered to the AMD 12 through an external charging pad 20 that is connected to the external charger 14. In some embodiments, the external charging pad 20 is configured to directly power the AMD 12 or it is configured to charge a rechargeable electrical power source 22 (FIGs. 4 and 6) of the medical device. The external charging pad 20 can be a hand-held device that is connected to the external charger 14, or it can be an internal component of the external charger. The external charger 14 and the charging pad 20 can also be integrated into a single device that is strapped on or attached to the patient with adhesive, and the like.

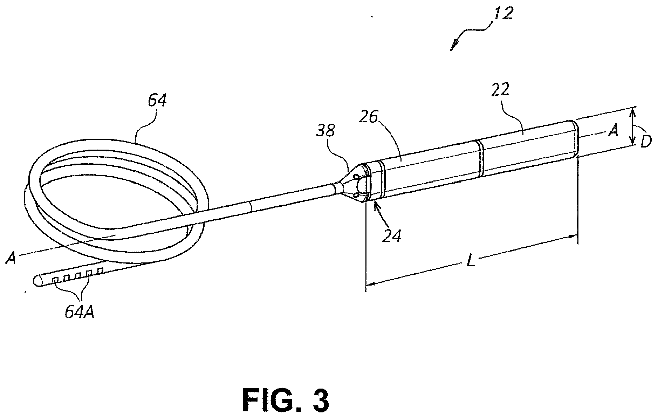

Referring now to FIG. 3, this drawing illustrates the AMD 12 as an exemplary embodiment of the various medical devices 100A to 100L illustrated in FIG. 1 and the exemplary AMD 12 shown in the medical device system 10 illustrated in FIG. 2 that can be implanted in a patient's body or worn externally on a patient's body. The AMD 12 is shown as an elongate device extending along a longitudinal axis A-A with an exemplary length "L" of about 33 mm and a width "W" of about 4 mm and comprising a feedthrough connector assembly 24 that is connected to the proximal end of a non-conductive housing 26.

The non-conductive housing 26 is preferably formed from a ceramic material that has a relatively high dielectric constant. An essentially pure alumina is a preferred ceramic material. The term "essentially pure alumina" refers to an alumina ceramic having the chemical formula Al₂O₃. "Essentially pure" means that the post-sintered alumina is at least 96% alumina. In a preferred embodiment, the post-sintered alumina is at least 99% high purity alumina. Moreover, as long as they are biocompatible and offer the appropriate mechanical robustness, other suitable non-conductive materials for the housing 26 include glass (e.g., high purity fused silica or HPFS) or plastic (e.g., PEEK).

Prior to sintering, alumina comprising the non-conductive housing 26 may be a paste, a slurry, or a green-state ceramic that is injection molded, powder pressed, and the like, into the desired shape of the housing 26 as a single monolithic structure. The green-state alumina ceramic is very pliable due to the organic binders and solvents that have been temporarily added to the system. As an assembly, the green-state ceramic housing 26 is then subjected to a controlled co-firing or co-sintering process in ambient air that comprises a binder bakeout portion, a sintering portion, and a cool down portion.

As shown in FIG. 4, the non-conductive housing 26 has a surrounding sidewall extending from an annular proximal rim 26A to an annular distal rim 26B. The proximal and distal rims 26A, 26B surround and define an open interior inside the housing 26. Proximal and distal weld rings 28 and 30, preferably made of titanium, are brazed to the respective proximal and distal annular rims 26A and 26B.

With respect to the proximal end of the non-conductive housing 26, to hermetically connect the weld ring 28 to the proximal rim 26A, the outwardly facing edge of the rim 26A is provided with a metallization (not shown). A suitable metallization comprises two metallization layers, a first adhesion layer that is directly applied to the outwardly facing edge of the rim 26A, and a second, wetting layer, which is applied on top of the adhesion layer. In a preferred embodiment, the adhesion layer is titanium, and the wetting layer is either molybdenum or niobium.

The adhesion and wetting metallization layers may be applied to the annular proximal rim 26A by thin and thick film technologies, such as printing, painting, plating, and deposition processes. Metallization processes include screen printing, pad printing, brush coating, direct bonding, active metal brazing, magnetron sputtering, physical vapor deposition, ion implantation, electroplating, and electroless plating. In an alternate embodiment, both the adhesion and wetting metallization layers may be provided by a single metallization layer. In a similar manner, the annular distal rim 26B is provided with a metallization. For the sake of simplicity, the adhesion and wetting layers contacted to both the proximal and distal rims 26A, 26B are intentionally not shown.

A braze pre-form, for example a gold ring-shaped preform, (not shown) is seated on each of the metallized rims 26A, 26B. The proximal weld ring 28/proximal gold-preform/ceramic housing 26/distal gold-preform/distal weld ring 30 assembly is then subjected to a brazing process, as is well known by those skilled in the art related to brazing a ceramic material to a metallic flange. The brazing process melts the gold to thereby form a hermetic seal joining the weld rings 28, 30 to the non-conductive housing 26 at the respective proximal and distal rims 26A, 26B.

The power source 22 shown in FIGs. 4 and 6 for the AMD 12 can be a capacitor or a rechargeable battery, for example a hermetically sealed rechargeable Li-ion battery. In that respect, the power source 22 is not limited to any one chemistry or even a rechargeable chemistry and can be of an alkaline cell, a primary lithium cell, a rechargeable lithium-ion cell, a Ni/cadmium cell, a Ni/metal hydride cell, a supercapacitor, a thin film solid-state cell, and the like. Preferably, the power source 22 is a lithium-ion electrochemical cell comprising a carbon-based or Li₄Ti₅O₁₂-based anode and a lithium metal oxide-based cathode, such as of LiCoO₂ or lithium nickel manganese cobalt oxide (LiNiₐMn_{b}Co_{1- a-b}O₂). The power source 22 can also be a solid-state thin film electrochemical cell having a lithium anode, a metal-oxide based cathode and a solid electrolyte, such as an electrolyte of LiPON (LiₓPO_{y}N_{z}). The power source 22 has outwardly extending opposite polarity terminals 22A and 22B that are configured to be electrically connected to a PCB assembly 34 housed inside the non-conductive housing 26. Titanium is a preferred material for the casing of the power source 22.

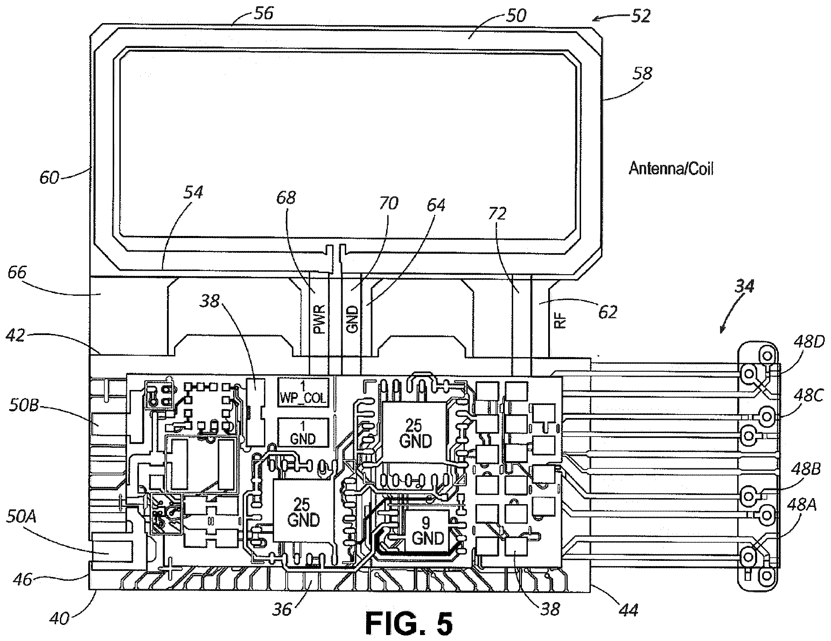

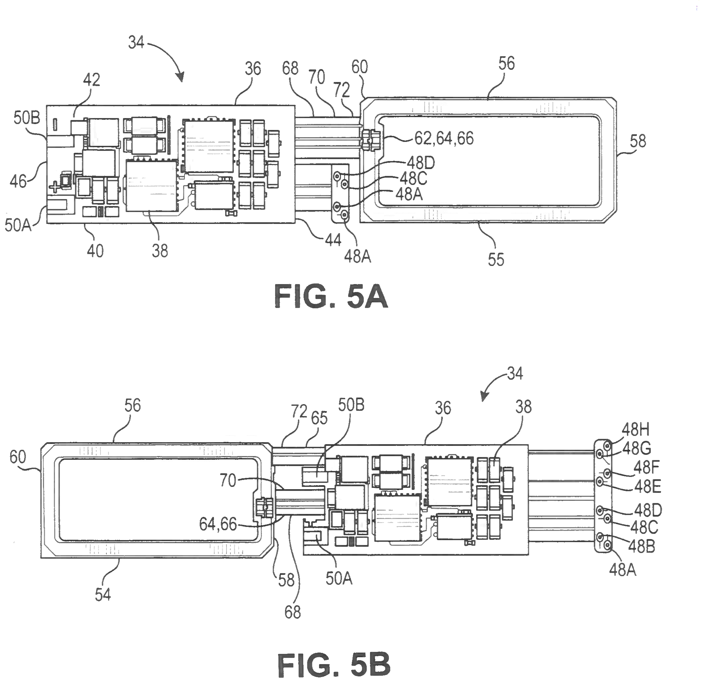

As shown in FIG. 5, the PCB assembly 34, which controls the various functions performed by the AMD 12, comprises a printed circuit board (PCB) 36 supporting at least one, and preferably a plurality of electronic components 38. The AMD functions include, but are not limited to, receiving sensed electrical signals pertaining to functions of the body tissue in which the AMD 12 is implanted and for delivering electrical current pulses to the body tissue.

The printed circuit board 36 has a length that comprises longitudinally extending right or upper and left or lower lateral edges 40 and 42 that meet with opposed proximal and distal ends 44 and 46. A plurality of electrical contacts 48A, 48B, 48C and 48D are supported on the PCB 36 at its proximal end or feedthrough connection end 44. While four electrical contacts 48A to 48D are shown in FIG. 5, it is within the scope of the present disclosure that less than and possibly many more contacts can be supported on the printed circuit board 36. The number of electrical contacts is dictated by the type of AND 12 and the medical condition it is designed to treat. Further, electrical contacts 50A and 50B are supported on the PCB 36 at its distal end or cell connection end 46. The proximal and distal electrical contacts 48A to 48D, 50A and 50B can be electrically conductive bond pads, solder bumps, circuit trace terminations, and the like.

In order to provide the AMD 12 as a device that can stay inside a patient's body for many years without needing to be replaced, a communication antenna/inductive charging coil assembly 52 is hingedly connected to the left lateral edge 42 of the PCB 36. The communication antenna/inductive charging coil assembly 52 has at least one to numerous winds of an electrically conductive wire or tape. The at least one, but preferably multiple turns of conductive wire or tape provide the communication antenna/inductive charging coil assembly 52 with a length that comprises longitudinally extending right or upper and left or lower coil lateral edges 54 and 56 that meet with opposed proximal and distal coil ends 58 and 60. The lengths of the lateral coil edges 54, 56 are substantially similar to the lengths of the lateral edges 40 and 42 of the PCB assembly 34. Similarly, the widths of the proximal and distal ends 58, 60 of the communication antenna/inductive charging coil assembly 52 are substantially similar to the widths of the opposed proximal and distal ends 44 and 46 of the PCB assembly 36.

After the distal weld ring 30 is hermetically connected to the distal rim 26B of the non-conductive housing 26, the power source terminals 22A, 22B are connected to the contacts pads 50A, 50B of the PCB assembly 34. Further, the power source 22 preferably has a third terminal pin (not shown) that is connected to the communication antenna/inductive charging coil assembly 52. The distal ring 30 of the housing 26 is then welded to the power source 22, preferably to a rim of the casing for the power source. In this position, the power source terminals 22A and 22B connected to the electrical contacts 50A and 50B of the PCB assembly 34 provide electrical power to the printed circuit board 36 and its electronic components 38.

FIGs. 5 and 6 illustrate that the communication antenna/inductive charging coil assembly 52 is hingedly connected to the left lateral edge 42 of the PCB assembly 34 by three flexible extensions 62, 64 and 66 from the printed circuit board 36. Preferably, the flexible extensions 62, 64 and 66 are integral living hinges, which are thin flexible hinge made from the same material as the PCB 36 to which they are connected. If desired, the hinges 62, 64 and 66 are thinner than the thickness of the main PCB 36 to allow the communication antenna/inductive charging coil assembly 52 to bend along the line of the hinges with respect to the PCB assembly 34. Further, it is within the scope of the present disclosure that the assembly 52 can also be hingedly connected to the opposed right lateral edge 44.

In an alternate embodiment, FIG. 5A illustrates that the communication antenna/inductive charging coil assembly 52 is hingedly connected to the proximal end or feedthrough connection end 44 of the PCB assembly 34 by a flexible extension 63 from the printed circuit board 36. Preferably, the flexible extension 63 is an integral living hinge which is made from the same material as the PCB 36 to which it is connected. Desirably, the hinge 63 is thinner than the thickness of the main PCB 36 to allow the communication antenna/inductive charging coil assembly 52 to bend along the line of the hinge with respect to the PCB assembly 34.

In another alternate embodiment, FIG. 5B illustrates that the communication antenna/inductive charging coil assembly 52 is hingedly connected to the distal end or cell connection end 46 of the PCB assembly 34 by spaced-apart flexible extensions 65 and 67 from the printed circuit board 36. Preferably, the flexible extensions 65 and 67 are integral living hinges which are made from the same material as the PCB 36 to which they are connected. Desirably, the hinges 65 and 67 are thinner than the thickness of the main PCB 36 to allow the communication antenna/inductive charging coil assembly 52 to bend along the line of the hinges with respect to the PCB assembly 34.

In that respect, what is important is that in its folded configuration, whether connected to the right lateral edge 40, the left lateral edge 42, the proximal end 44 or the distal end 46, the communication antenna/inductive charging coil assembly 52 is positioned directly spaced above or directly spaced below the PCB assembly 36. Thus, it is an aspect of the present AMD 12 that the communication antenna/inductive charging coil assembly 52 is hingedly moved into a position that is aligned directly spaced above or directly spaced below the PCB assembly 34. In its folded and aligned configuration, the lateral edges 54 and 56 of the communication antenna/inductive charging coil assembly 52 are aligned directly spaced above or directly spaced below the respective lateral edges 42 and 40 of the PCB assembly 34, and the proximal and distal coil ends 58 and 60 are aligned directly spaced above or directly spaced below the respective proximal and distal ends 44 and 46 of the PCB assembly 34.

Moreover, while the communication antenna/inductive charging coil assembly 52 is shown in the drawings as a substantially rectangular structure, that is exemplary. In alternative embodiments, the communication antenna/inductive charging coil assembly 52 has an oval shape, a circular shape or a multi-sided shape that is neither rectangular or circular but has both straight sides and curved sides. Additionally, the communication antenna/inductive charging coil assembly 52 need not have a length and a width that substantially aligns with the length and width of the PCB assembly 34. In those alternate embodiments, the communication antenna/inductive charging coil assembly 52 has a length or width that is either greater or less than that of the PCB assembly 34.

The communication antenna/inductive charging coil assembly 52 is connected to the electronic circuits 38 supported on the PCB 36 by a power trace 68, a ground trace 70 and an RF signal trace 72. Trace 72 is configured to carry an RF signal from the communication antenna/inductive charging coil assembly 52 to a dedicated circuit or circuits on the PCB assembly 36 that are configured to convert RF or inductive energy signals received from the external charging pad 20 connected to the external charger 14 (FIG. 2) into a direct current voltage to charge the power source 22 to power the electronic components 38 of the PCB assembly 34. The communication antenna/inductive charging coil assembly 52 is also configured to receive communication signals from the patient and clinician programmers 16, 18 and send communication signals from the medical device 12 back to the programmers 16 and 18 related to electrical stimulation of body tissue, sensed biological signals from body tissue, or both stimulate and sense RF signals.

In that respect, the communication antenna/inductive charging coil assembly 52 may be used to receive both charging signals (e.g., inductive energy in the 13.56 MHz ISM band) and telemetry signals (e.g., Bluetooth LE or BLE). Thus, the PCB assembly 34 includes electronic components that allow charging signals in the 13.56 MHz ISM band to pass through but will reject signals outside the 13.56 MHz ISM band while charging the electrical power source 22, including telemetry signals in the 2.45 GHz ISM band (BLE). Moreover, the electronic components 38 of the PCB assembly 34 allow telemetry signals to pass when the AMD 12 is communicating with an external device, such as the patient and clinician programmers 16 and 18.

As already described, the power source 22 provides electrical power to the PCB assembly 34 housed inside the non-conductive housing 26 to power the electronic components 38 supported on its printed circuit board 36. The PCB assembly 34 in turn provides electrical power to the electrodes 74A of a lead 74 extending distally from a strain-relief device 76 that is detachably connected to the feedthrough connector assembly 24 which, in turn, is connected to the proximal weld ring 28 of the non-conductive housing 26.

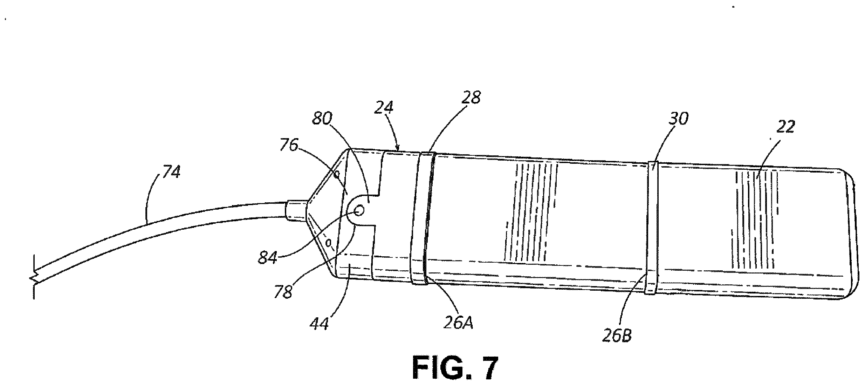

In particular, the strain-relief device 76 has an inlet 76A extending inwardly from an annular sidewall into a beveled sidewall. The inlet 76A has a threaded opening 78. The inlet 76A of the strain-relief device 76 is sized to receive a hub 80 (FIGs. 4 and 7) extending outwardly from the ferrule 44 of the feedthrough 24. The hub 80 also has a threaded opening 82 so that with the feedthrough hub 80 positioned in the inlet 76A of the strain-relief device 76, the respective threaded openings 78, 82 are aligned with each other. A threaded member 84 is then threaded into the aligned openings 78 and 82 to fasten the feedthrough connector assembly 24 to the strain-relief device 76. The feedthrough connector assembly 24 has a number of terminal pins (not shown) that are hermetically sealed in an electrically non-conductive insulator supported in an opening in the ferrule 44 for the feedthrough, as is well known by those skilled in the art of feedthrough assemblies. Suitable feedthrough connector assemblies 24 configured to detachably connect to the strain-relief device 76 for the lead 74 are described in U.S. Pub. Nos. 2023/0372720, 2023/0372721, 2023/0405312, and 2024/0374905,

As previously described, the lead 74 extends distally from the strain-relief device 76, which is detachably connected to the feedthrough connector assembly 24 connected to the non-conductive housing 26 for the PCB assembly 34. The lead 74 has a number of electrodes 74A that are configured to deliver current pulses to body tissue, receive sensed electrical signals pertaining to functions of body tissue in which the AMD 12 is implanted or on which the AMD 12 is externally supported, or both sense electrical signals and deliver current pulses.

Thus, the elongated shape of the AMD 12 allows the implantation procedure to be performed by injecting or inserting the AMD into body tissue using of an insertion tool, typically through a very small incision. With the AMD 12 implanted in body tissue, the electrical power source 22 electrically connected to the PCB assembly 34 provides electrical power to the electrodes 74A of the lead 74. The electrodes 74A are configured to electrically stimulate body tissue or sense biological signals, or sense and stimulate.

## Claims

1. An active medical device (AMD) (12), comprising:
a) a ceramic housing (26), comprising:
i) an annular sidewall defining an open housing extending from an annular proximal housing rim (26A) at a proximal open end of the housing (26) to an annular distal housing rim (26B) at a distal open end of the housing (26);
ii) a proximal metallic ring (28) brazed to the proximal housing rim (26A); and
iii) a distal metallic ring (30) brazed to the distal housing rim (26B);
b) a printed circuit board (PCB) assembly (34) housed inside the ceramic housing (26) and comprising a printed circuit board (36) supporting at least one electronic component (38), the printed circuit board (36) comprising longitudinally extending right and left lateral edges (40,42) meeting opposed proximal and distal ends (44,46), wherein the proximal end (44) of the printed circuit board (36) supports a plurality of proximal electrical contacts (48A-D), and the distal end (46) of the printed circuit board (36) supports at least two distal electrical contacts (50A-B);
c) a communication antenna/inductive charging coil assembly (52) connected to one of the right and left lateral edges (40,42) of the printed circuit board (36) by a hinge (62,64,66) and being electrically connected to the at least one electronic component (38) of the PCB assembly (34), wherein, in a folded configuration at the hinge (62,64,66), the communication antenna/inductive charging coil assembly (52) is housed inside the ceramic housing (26), spaced above the PCB assembly (34);
d) an electrical power source (22) comprising a metallic casing, wherein the electrical power source (22) has opposite polarity terminals (22A-B) that are connected to the at least two distal electrical contacts (50A-B) at the distal end (46) of the printed circuit board (36), and wherein the distal metallic ring (30) at the distal housing rim (26B) of the ceramic housing (26) is welded to the power source casing adjacent to the distal electrical contacts (50A-B) of the printed circuit board (36) connected to the opposite polarity terminals (22A-B) of the electrical power source (22); and
e) a feedthrough connector assembly (24) comprising an electrically non-conductive insulator brazed to a ferrule (44) in a ferrule opening, wherein the insulator supports a plurality of hermetically sealed terminal pins comprising terminal pin device side ends extending outwardly beyond an insulator device side and terminal pin body fluid side ends extending outwardly beyond an insulator body fluid side, and wherein the device side ends of the terminal pins are electrically connected to the plurality of proximal electrical contacts (48A-D) at the proximal end (44) of the printed circuit board (36) adjacent to the proximal housing rim (26A) of the ceramic housing (26) brazed to the proximal metallic ring (28) connected to the ferrule (44) of the feedthrough connector assembly (24).

2. The AMD of claim 1, wherein:
a) the communication antenna/inductive charging coil assembly (52) comprises at least one turn of conductive wire or tape having a length that comprises longitudinally extending coil right and left lateral edges (54,56) meeting opposed coil proximal and distal ends (58,60) so that, in the folded configuration at the hinge (62,64,66) connected to one of the right and left lateral edges (40,42) of the printed circuit board (36), the coil right and left lateral edges (54,56) are aligned directly spaced above the respective right and left lateral edges (40,42) and the opposed coil proximal and distal ends (58,60) are aligned directly spaced above the respective proximal and distal ends (44,46) of the printed circuit board;
b) the device side ends of the terminal pins electrically connected to the plurality of proximal electrical contacts (48A-D) at the proximal end (44) of the printed circuit board (36) are spaced below the proximal end (58) of the communication antenna/inductive charging coil assembly (52), adjacent to the proximal metallic ring (28) of the ceramic housing (26) connected to the ferrule (44) of the feedthrough connector assembly (24); and
c) the opposite polarity terminals (22A-B) of the power source (22) connected to the at least two distal electrical contacts (50A-B) at the distal end (46) of the printed circuit board (36) are spaced below the distal end (60) of the communication antenna/inductive charging coil assembly (52), adjacent to the distal metallic ring (30) of the ceramic housing (26) connected to the power source casing.

3. The AMD of claim 1 or claim 2, wherein the hinge (62,64,66) connected to one of the right and left lateral edges (40,42) of the printed circuit board (36) is a flexible extension from the printed circuit board (36) of the PCB assembly (34).

4. The AMD of any preceding claim, wherein the printed circuit board (36) has a first thickness adjacent to the communication antenna/inductive charging coil assembly (52), and wherein the hinge (62,64,66) connected to one of the right and left lateral edges (40,42) of the printed circuit board (36) has a second thickness that is less than the first thickness.

5. The AMD of any preceding claim, wherein the hinge (62,64,66) comprises at least two spaced-apart flexible extensions (62,64,66) from one of the right and left lateral edges (40,42) of the printed circuit board (36).

6. The AMD of any preceding claim, wherein the feedthrough connector assembly (24) is connectable to a lead (74), and preferably wherein a proximal end of the lead (74) is connected to a strain-relief device (76) and the strain-relief device (76) is connectable to the feedthrough connector assembly (24).

7. The AMD of any preceding claim, wherein the power source (22) is rechargeable, and preferably wherein the communication antenna/inductive charging coil assembly (52) comprises a charging coil connected to a charging circuit on the printed circuit board (36), and wherein the charging circuit is configured to convert RF or inductive energy received from the charging coil into a direct current voltage to charge the power source (22) to power the PCB assembly (34).

8. An active medical device (AMD) (12), comprising:
a) a ceramic housing (26), comprising:
i) an annular sidewall defining an open housing extending from an annular proximal housing rim (26A) at a proximal open end of the housing (26) to an annular distal housing rim (26B) at a distal open end of the housing (26);
ii) a proximal metallic ring (28) brazed to the proximal housing rim (26A); and
iii) a distal metallic ring (30) brazed to the distal housing rim (26B);
b) a printed circuit board (PCB) assembly (34) housed inside the ceramic housing (26) and comprising a printed circuit board (36) supporting at least one electronic component (38), the printed circuit board (36) comprising longitudinally extending right and left lateral edges (40,42) meeting opposed proximal and distal ends (44,46), wherein the proximal end (44) of the printed circuit board (36) supports a plurality of proximal electrical contacts (48A-D), and the distal end (46) of the printed circuit board (36) supports at least two distal electrical contacts (50A-B);
c) a communication antenna/inductive charging coil assembly (52) connected to one of the proximal and distal ends (44,46) of the printed circuit board (36) by a hinge (62,64,66) and being electrically connected to the at least one electronic component (38) of the PCB assembly (34), wherein, in a folded configuration at the hinge (62,64,66), the communication antenna/inductive charging coil assembly (52) is housed inside the ceramic housing (26), spaced above the PCB assembly (34);
d) an electrical power source (22) comprising a metallic casing, wherein the electrical power source (22) has opposite polarity terminals (22A-B) that are connected to the at least two distal electrical contacts (50A-B) at the distal end (46) of the printed circuit board (36), and wherein the distal metallic ring (30) at the distal open end of the ceramic housing (26) is welded to the power source casing adjacent to the distal electrical contacts (50A-B) of the printed circuit board (26) connected to the opposite polarity terminals (22A-B) of the electrical power source (22); and
e) a feedthrough connector assembly (24) comprising an electrically non-conductive insulator brazed to a ferrule (44) in a ferrule opening, wherein the insulator supports a plurality of hermetically sealed terminal pins comprising terminal pin device side ends extending outwardly beyond an insulator device side and terminal pin body fluid side ends extending outwardly beyond an insulator body fluid side, and wherein the device side ends of the terminal pins are electrically connected to the plurality of proximal electrical contacts (48A-D) at the proximal end (44) of the printed circuit board (36) adjacent to the proximal housing rim (26A) of the ceramic housing (26) brazed to the proximal ring (28) connected to the ferrule (44) of the feedthrough connector assembly (24).

9. The AMD of claim 8, wherein:
a) the communication antenna/inductive charging coil assembly (52) comprises at least one turn of conductive wire or tape having a length that comprises longitudinally extending coil right and left lateral edges (54,56) meeting opposed coil proximal and distal ends (58,60) so that, in the folded configuration at the hinge (62,64,66) connected to one of the proximal and distal ends (44,46) of the printed circuit board (36), the coil right and left lateral edges (54,56) are aligned directly spaced above the respective right and left lateral edges (40,42) and the opposed coil proximal and distal ends (58,60) are aligned directly spaced above the respective proximal and distal ends (44,46) of the printed circuit board (36);
b) the device side ends of the terminal pins electrically connected to the plurality of proximal electrical contacts (48A-D) at the proximal end (44) of the printed circuit board (36) are spaced below the proximal end (58) of the communication antenna/inductive charging coil assembly (52), adjacent to the proximal metallic ring (28) of the ceramic housing (26) connected to the ferrule (44) of the feedthrough connector assembly (24); and
c) the opposite polarity terminals (22A-B) of the power source (22) connected to the at least two distal electrical contacts (50A-B) at the distal end (46) of the printed circuit board (36) are spaced below the distal end (60) of the communication antenna/inductive charging coil assembly (52), adjacent to the distal metallic ring (30) of the ceramic housing (26) connected to the power source casing.

10. The AMD of claim 8 or claim 9, wherein the hinge (62,64,66) connecting one of the proximal and distal ends (44,46) of the printed circuit board (36) to the communication antenna/inductive charging coil assembly (52) is a living hinge.

11. The AMD of any of claims 8 to 10, wherein the hinge (62,64,66) connected to one of the proximal and distal ends (44,46) of the printed circuit board (36) is a flexible extension from the printed circuit board (36) of the PCB assembly (34), or wherein the hinge (62,64,66) comprises at least two spaced-apart flexible extensions (62,64,66) from one of the proximal and distal ends (44,46) of the printed circuit board (36).

12. The AMD of any of claims 8 to 11, wherein the printed circuit board (36) has a first thickness adjacent to the communication antenna/inductive charging coil assembly (52), and the hinge (62,64,66) connected to one of the proximal and distal ends (44,46) of the printed circuit board (36) has a second thickness that is less than the first thickness.

13. The AMD of any of claims 8 to 12, wherein the feedthrough connector assembly (24) is connectable to a lead (74), and preferably wherein a proximal end of the lead (74) is connected to a strain-relief device (76) and the strain-relief device (76) is connectable to the feedthrough connector assembly (24).

14. The AMD of any of claims 8 to 13, wherein the power source (22) is rechargeable, and preferably wherein the communication antenna/inductive charging coil assembly (52) comprises a charging coil connected to a charging circuit on the printed circuit board (36), and wherein the charging circuit is configured to convert RF or inductive energy received from the charging coil into a direct current voltage to charge the power source (22) to power the PCB assembly (34).

15. The AMD of any of claims 8 to 14, wherein the communication antenna/inductive charging coil assembly (52) has a shaped selected from a rectangular shape, an oval shape, a circular shape, a multi-sided shape that is neither rectangular nor circular but has both straight sides and curved sides, and wherein the communication antenna/inductive charging coil assembly (52) either has or does not have a length and a width that substantially aligns with a length and a width of the PCB assembly (34).

## Patentansprüche

1. Aktive medizinische Vorrichtung (AMD) (12), umfassend:
a) ein Keramikgehäuse (26), umfassend:
i) eine ringförmige Seitenwand, die ein offenes Gehäuse definiert, das sich von einem ringförmigen proximalen Gehäuserand (26A) an einem proximalen offenen Ende des Gehäuses (26) bis zu einem ringförmigen distalen Gehäuserand (26B) an einem distalen offenen Ende des Gehäuses (26) erstreckt;
ii) einen proximalen metallischen Ring (28), der mit dem proximalen Gehäuserand (26A) verlötet ist; und
iii) einen distalen metallischen Ring (30), der mit dem distalen Gehäuserand (26B) verlötet ist;
b) eine Leiterplatten(PCB)-Baugruppe (34), die im Keramikgehäuse (26) aufgenommen ist und eine Leiterplatte (36) umfasst, die mindestens ein elektronisches Bauteil (38) trägt, wobei die Leiterplatte (36) rechte und linke Seitenkanten (40, 42) umfasst, die sich in Längsrichtung erstrecken und an gegenüberliegenden proximalen und distalen Enden (44, 46) zusammenlaufen, wobei das proximale Ende (44) der Leiterplatte (36) eine Vielzahl von proximalen elektrischen Kontakten (48A-D) trägt, und das distale Ende (46) der Leiterplatte (36) mindestens zwei distale elektrische Kontakte (50A-B) trägt;
c) eine Kommunikationsantennen-/induktive Ladespulen-Baugruppe (52), die über ein Scharnier (62, 64, 66) mit einer von der rechten und linken Seitenkante (40, 42) der Leiterplatte (36) verbunden ist und mit dem mindestens einen elektronischen Bauteil (38) der PCB-Baugruppe (34) elektrisch verbunden ist, wobei in einer zusammengeklappten Konfiguration am Scharnier (62, 64, 66) die Kommunikationsantennen-/induktive Ladespulen-Baugruppe (52) innerhalb des Keramikgehäuses (26) mit Abstand über der PCB-Baugruppe (34) aufgenommen ist;
d) eine elektrische Energiequelle (22), umfassend eine metallische Hülle, wobei die elektrische Energiequelle (22) Anschlüsse (22A-B) mit entgegengesetzter Polarität aufweist, die mit den mindestens zwei distalen elektrischen Kontakten (50A-B) am distalen Ende (46) der Leiterplatte (36) verbunden sind, und wobei der distale metallische Ring (30) am distalen Gehäuserand (26B) des Keramikgehäuses (26) an die Hülle der Energiequelle benachbart an die distalen elektrischen Kontakte (50A-B) der Leiterplatte (36) angeschweißt ist, die mit den Anschlüssen (22A-B) mit entgegengesetzter Polarität der elektrischen Energiequelle (22) verbunden sind; und
e) eine Durchführungsverbinder-Baugruppe (24), die einen elektrisch nichtleitenden Isolator umfasst, der in einer Hülsenöffnung an eine Hülse (44) gelötet ist, wobei der Isolator eine Vielzahl von hermetisch abgedichteten Anschlussstiften trägt, die vorrichtungsseitige Enden der Anschlussstifte, die sich über eine vorrichtungsseitige Seite des Isolators hinaus nach außen erstrecken, und körperflüssigkeitsseitige Enden der Anschlussstifte, die sich über eine Körperflüssigkeitsseite des Isolators hinaus nach außen erstrecken, umfassen, und wobei die vorrichtungsseitigen Enden der Anschlussstifte mit der Vielzahl von proximalen elektrischen Kontakten (48A-D) am proximalen Ende (44) der Leiterplatte (36) benachbart zum proximalen Gehäuserand (26A) des Keramikgehäuses (26) elektrisch verbunden sind, das an den proximalen metallischen Ring (28) gelötet ist, der mit der Hülse (44) der Durchführungsverbinder-Baugruppe (24) verbunden ist.

2. AMD nach Anspruch 1, wobei:
a) die Kommunikationsantennen-/induktive Ladespulen-Baugruppe (52) mindestens eine Windung aus leitfähigem Draht oder Band mit einer Länge umfasst, die sich in Längsrichtung erstreckende rechte und linke Seitenkanten (54, 56) der Spule umfasst, die an gegenüberliegenden proximalen und distalen Enden (58, 60) der Spule zusammenlaufen, sodass in der zusammengeklappten Konfiguration am Scharnier (62, 64, 66), das mit einer von der rechten und linken Seitenkante (40, 42) der Leiterplatte (36) verbunden ist, die rechte und linke Seitenkante (54, 56) der Spule direkt mit Abstand über der jeweiligen rechten und linken Seitenkante (40, 42) ausgerichtet sind und die gegenüberliegenden proximalen und distalen Enden (58, 60) der Spule direkt mit Abstand über dem jeweiligen proximalen und distalen Ende (44, 46) der Leiterplatte ausgerichtet sind;
b) die vorrichtungsseitigen Enden der Anschlussstifte, die elektrisch mit der Vielzahl von proximalen elektrischen Kontakten (48A-D) am proximalen Ende (44) der Leiterplatte (36) verbunden sind, mit Abstand unter dem proximalen Ende (58) der Kommunikationsantennen-/induktive Ladespulen-Baugruppe (52) liegen, benachbart an den proximalen metallischen Ring (28) des Keramikgehäuses (26), der mit der Hülse (44) der Durchführungsverbinder-Baugruppe (24) verbunden ist; und
c) die Anschlüsse (22A-B) mit entgegengesetzter Polarität der Energiequelle (22), die mit den mindestens zwei distalen elektrischen Kontakten (50A-B) am distalen Ende (46) der Leiterplatte (36) verbunden sind, mit Abstand unter dem distalen Ende (60) der Kommunikationsantennen-/induktive Ladespulen-Baugruppe (52) liegen, benachbart an den distalen metallischen Ring (30) des Keramikgehäuses (26), das mit der Hülle der Energiequelle verbunden ist.

3. AMD nach Anspruch 1 oder Anspruch 2, wobei das Scharnier (62, 64, 66), das mit einer von der rechten und linken Seitenkante (40, 42) der Leiterplatte (36) verbunden ist, eine flexible Verlängerung der Leiterplatte (36) der PCB-Baugruppe (34) darstellt.

4. AMD nach einem der vorhergehenden Ansprüche, wobei die Leiterplatte (36) eine erste Dicke benachbart zu der Kommunikationsantennen-/induktive Ladespulen-Baugruppe (52) aufweist und wobei das Scharnier (62, 64, 66), das mit einer von der rechten und linken Seitenkante (40, 42) der Leiterplatte (36) verbunden ist, eine zweite Dicke aufweist, die geringer ist als die erste Dicke.

5. AMD nach einem der vorhergehenden Ansprüche, wobei das Scharnier (62, 64, 66) mindestens zwei voneinander beabstandete flexible Verlängerungen (62, 64, 66) von einer von der rechten und linken Seitenkante (40, 42) der Leiterplatte (36) umfasst.

6. AMD nach einem der vorhergehenden Ansprüche, wobei die Durchführungsverbinder-Baugruppe (24) mit einer Leitung (74) verbindbar ist und vorzugsweise wobei ein proximales Ende der Leitung (74) mit einer Zugentlastungsvorrichtung (76) verbunden ist und die Zugentlastungsvorrichtung (76) mit der Durchführungsverbinder-Baugruppe (24) verbindbar ist.

7. AMD nach einem der vorhergehenden Ansprüche, wobei die Energiequelle (22) wiederaufladbar ist und vorzugsweise wobei die Kommunikationsantennen-/induktive Ladespulen-Baugruppe (52) eine Ladespule umfasst, die mit einer Ladeschaltung auf der Leiterplatte (36) verbunden ist, und wobei die Ladeschaltung dazu konfiguriert ist, von der Ladespule empfangene HF- oder induktive Energie in eine Gleichspannung umzuwandeln, um die Energiequelle (22) zum Betreiben der PCB-Baugruppe (34) aufzuladen.

8. Aktive medizinische Vorrichtung (AMD) (12), umfassend:
a) ein Keramikgehäuse (26), umfassend:
i) eine ringförmige Seitenwand, die ein offenes Gehäuse definiert, das sich von einem ringförmigen proximalen Gehäuserand (26A) an einem proximalen offenen Ende des Gehäuses (26) bis zu einem ringförmigen distalen Gehäuserand (26B) an einem distalen offenen Ende des Gehäuses (26) erstreckt;
ii) einen proximalen metallischen Ring (28), der mit dem proximalen Gehäuserand (26A) verlötet ist; und
iii) einen distalen metallischen Ring (30), der mit dem distalen Gehäuserand (26B) verlötet ist;
b) eine Leiterplatten(PCB)-Baugruppe (34), die im Keramikgehäuse (26) aufgenommen ist und eine Leiterplatte (36) umfasst, die mindestens ein elektronisches Bauteil (38) trägt, wobei die Leiterplatte (36) rechte und linke Seitenkanten (40, 42) umfasst, die sich in Längsrichtung erstrecken und an gegenüberliegenden proximalen und distalen Enden (44, 46) zusammenlaufen, wobei das proximale Ende (44) der Leiterplatte (36) eine Vielzahl von proximalen elektrischen Kontakten (48A-D) trägt, und das distale Ende (46) der Leiterplatte (36) mindestens zwei distale elektrische Kontakte (50A-B) trägt;
c) eine Kommunikationsantennen-/induktive Ladespulen-Baugruppe (52), die über ein Scharnier (62, 64, 66) mit einem von dem proximalen und distalen Ende (44, 46) der Leiterplatte (36) verbunden ist und mit dem mindestens einen elektronischen Bauteil (38) der PCB-Baugruppe (34) elektrisch verbunden ist, wobei in einer zusammengeklappten Konfiguration am Scharnier (62, 64, 66) die Kommunikationsantennen-/induktive Ladespulen-Baugruppe (52) innerhalb des Keramikgehäuses (26) mit Abstand über der PCB-Baugruppe (34) aufgenommen ist;
d) eine elektrische Energiequelle (22), umfassend eine metallische Hülle, wobei die elektrische Energiequelle (22) Anschlüsse (22A-B) mit entgegengesetzter Polarität aufweist, die mit den mindestens zwei distalen elektrischen Kontakten (50A-B) am distalen Ende (46) der Leiterplatte (36) verbunden sind, und wobei der distale metallische Ring (30) am distalen offenen Ende des Keramikgehäuses (26) an die Hülle der Energiequelle benachbart an die distalen elektrischen Kontakte (50A-B) der Leiterplatte (26) angeschweißt ist, die mit den Anschlüssen (22A-B) mit entgegengesetzter Polarität der elektrischen Energiequelle (22) verbunden sind; und
e) eine Durchführungsverbinder-Baugruppe (24), die einen elektrisch nichtleitenden Isolator umfasst, der in einer Hülsenöffnung an eine Hülse (44) gelötet ist, wobei der Isolator eine Vielzahl von hermetisch abgedichteten Anschlussstiften trägt, die vorrichtungsseitige Enden der Anschlussstifte, die sich über eine vorrichtungsseitige Seite des Isolators hinaus nach außen erstrecken, und körperflüssigkeitsseitige Enden der Anschlussstifte, die sich über eine Körperflüssigkeitsseite des Isolators hinaus nach außen erstrecken, umfassen, und wobei die vorrichtungsseitigen Enden der Anschlussstifte mit der Vielzahl von proximalen elektrischen Kontakten (48A-D) am proximalen Ende (44) der Leiterplatte (36) benachbart zum proximalen Gehäuserand (26A) des Keramikgehäuses (26) elektrisch verbunden sind, das an den proximalen Ring (28) gelötet ist, der mit der Hülse (44) der Durchführungsverbinder-Baugruppe (24) verbunden ist.

9. AMD nach Anspruch 8, wobei:
a) die Kommunikationsantennen-/induktive Ladespulen-Baugruppe (52) mindestens eine Windung aus leitfähigem Draht oder Band mit einer Länge umfasst, die sich in Längsrichtung erstreckende rechte und linke Seitenkanten (54, 56) der Spule umfasst, die an gegenüberliegenden proximalen und distalen Enden (58, 60) der Spule zusammenlaufen, sodass in der zusammengeklappten Konfiguration am Scharnier (62, 64, 66), das mit einem von dem proximalen und distalen Ende (44, 46) der Leiterplatte (36) verbunden ist, die rechte und linke Seitenkante (54, 56) der Spule direkt mit Abstand über der jeweiligen rechten und linken Seitenkante (40, 42) ausgerichtet sind und die gegenüberliegenden proximalen und distalen Enden (58, 60) der Spule direkt mit Abstand über dem jeweiligen proximalen und distalen Ende (44, 46) der Leiterplatte (36) ausgerichtet sind;
b) die vorrichtungsseitigen Enden der Anschlussstifte, die elektrisch mit der Vielzahl von proximalen elektrischen Kontakten (48A-D) am proximalen Ende (44) der Leiterplatte (36) verbunden sind, mit Abstand unter dem proximalen Ende (58) der Kommunikationsantennen-/induktive Ladespulen-Baugruppe (52) liegen, benachbart an den proximalen metallischen Ring (28) des Keramikgehäuses (26), der mit der Hülse (44) der Durchführungsverbinder-Baugruppe (24) verbunden ist; und
c) die Anschlüsse (22A-B) mit entgegengesetzter Polarität der Energiequelle (22), die mit den mindestens zwei distalen elektrischen Kontakten (50A-B) am distalen Ende (46) der Leiterplatte (36) verbunden sind, mit Abstand unter dem distalen Ende (60) der Kommunikationsantennen-/induktive Ladespulen-Baugruppe (52) liegen, benachbart an den distalen metallischen Ring (30) des Keramikgehäuses (26), das mit der Hülle der Energiequelle verbunden ist.

10. AMD nach Anspruch 8 oder Anspruch 9, wobei das Scharnier (62, 64, 66), das eines von dem proximalen und distalen Ende (44, 46) der Leiterplatte (36) mit der Kommunikationsantennen-/induktive Ladespulen-Baugruppe (52) verbindet, ein Filmscharnier ist.

11. AMD nach einem der Ansprüche 8 bis 10, wobei das Scharnier (62, 64, 66), das mit einem von dem proximalen und dem distalen Ende (44, 46) der Leiterplatte (36) verbunden ist, eine flexible Verlängerung der Leiterplatte (36) der PCB-Baugruppe (34) ist, oder wobei das Scharnier (62, 64, 66) mindestens zwei voneinander beabstandete flexible Verlängerungen (62, 64, 66) von einem von dem proximalen und distalen Ende (44, 46) der Leiterplatte (36) umfasst.

12. AMD nach einem der Ansprüche 8 bis 11, wobei die Leiterplatte (36) eine erste Dicke benachbart zu der Kommunikationsantennen-/induktive Ladespulen-Baugruppe (52) aufweist und das Scharnier (62, 64, 66), das mit einem von dem proximalen und distalen Ende (44, 46) der Leiterplatte (36) verbunden ist, eine zweite Dicke aufweist, die geringer ist als die erste Dicke.

13. AMD nach einem der Ansprüche 8 bis 12, wobei die Durchführungsverbinder-Baugruppe (24) mit einer Leitung (74) verbindbar ist und vorzugsweise wobei ein proximales Ende der Leitung (74) mit einer Zugentlastungsvorrichtung (76) verbunden ist und die Zugentlastungsvorrichtung (76) mit der Durchführungsverbinder-Baugruppe (24) verbindbar ist.

14. AMD nach einem der Ansprüche 8 bis 13, wobei die Energiequelle (22) wiederaufladbar ist und vorzugsweise wobei die Kommunikationsantennen-/induktive Ladespulen-Baugruppe (52) eine Ladespule umfasst, die mit einer Ladeschaltung auf der Leiterplatte (36) verbunden ist, und wobei die Ladeschaltung dazu konfiguriert ist, von der Ladespule empfangene HF- oder induktive Energie in eine Gleichspannung umzuwandeln, um die Energiequelle (22) zum Betreiben der PCB-Baugruppe (34) aufzuladen.

15. AMD nach einem der Ansprüche 8 bis 14, wobei die Kommunikationsantennen-/induktive Ladespulen-Baugruppe (52) eine Form aufweist, die aus einer rechteckigen Form, einer ovalen Form, einer kreisförmigen Form oder einer vielseitigen Form, die weder rechteckig noch kreisförmig ist, sondern sowohl gerade Seiten als auch gekrümmte Seiten aufweist, ausgewählt ist, und wobei die Kommunikationsantennen-/induktive Ladespulen-Baugruppe (52) entweder eine Länge und eine Breite aufweist oder nicht aufweist, die im Wesentlichen mit einer Länge und einer Breite der PCB-Baugruppe (34) ausgerichtet sind.

## Revendications

1. Dispositif médical actif (DMA) (12), comprenant :
a) un logement en céramique (26), comprenant :
i) une paroi latérale annulaire définissant un logement ouvert s'étendant d'un rebord de logement proximal annulaire (26A) au niveau d'une extrémité ouverte proximale du logement (26) à un rebord de logement distal annulaire (26B) au niveau d'une extrémité ouverte distale du logement (26) ;
ii) une bague métallique proximale (28) brasée sur le rebord de logement proximal (26A) ; et
iii) une bague métallique distale (30) brasée sur le rebord de logement distal (26B) ;
b) un ensemble de carte de circuit imprimé (PCB) (34) logé à l'intérieur du logement en céramique (26) et comprenant une carte de circuit imprimé (36) supportant au moins un composant électronique (38), la carte de circuit imprimé (36) comprenant des bords latéraux droit et gauche s'étendant de manière longitudinale (40, 42) rencontrant des extrémités opposées proximale et distale (44, 46), dans lequel l'extrémité proximale (44) de la carte de circuit imprimé (36) supporte une pluralité de contacts électriques proximaux (48A-D), et l'extrémité distale (46) de la carte de circuit imprimé (36) supporte au moins deux contacts électriques distaux (50A-B) ;
c) un ensemble d'antenne de communication/de bobine de charge par induction (52) connecté à l'un des bords latéraux droit et gauche (40, 42) de la carte de circuit imprimé (36) par une charnière (62, 64, 66) et étant connecté électriquement à l'au moins un composant électronique (38) de l'ensemble de PCB (34), dans lequel, dans une configuration pliée au niveau de la charnière (62, 64, 66), l'ensemble d'antenne de communication/de bobine de charge par induction (52) est logé à l'intérieur du logement en céramique (26), espacé au-dessus de l'ensemble de PCB (34) ;
d) une source de puissance électrique (22) comprenant un boîtier métallique, dans lequel la source de puissance électrique (22) a des bornes de polarité opposée (22A-B) qui sont connectées aux au moins deux contacts électriques distaux (50A-B) au niveau de l'extrémité distale (46) de la carte de circuit imprimé (36), et dans lequel la bague métallique distale (30) au niveau du rebord de logement distal (26B) du logement en céramique (26) est soudée au boîtier de source de puissance adjacent aux contacts électriques distaux (50A-B) de la carte de circuit imprimé (36) connectés aux bornes de polarité opposée (22A-B) de la source de puissance électrique (22) ; et
e) un ensemble de connecteur de traversée (24) comprenant un isolateur non conducteur électriquement brasé sur une férule (44) dans une ouverture de férule, dans lequel l'isolateur supporte une pluralité de broches de borne scellées hermétiquement comprenant des extrémités côté dispositif de broche de borne s'étendant vers l'extérieur au-delà d'un côté dispositif d'isolateur et des extrémités côté fluide corporel de broche de borne s'étendant vers l'extérieur au-delà d'un côté fluide corporel d'isolateur, et dans lequel les extrémités côté dispositif des broches de borne sont connectées électriquement à la pluralité de contacts électriques proximaux (48A-D) au niveau de l'extrémité proximale (44) de la carte de circuit imprimé (36) adjacente au rebord de logement proximal (26A) du logement en céramique (26) brasé sur la bague métallique proximale (28) connectée à la férule (44) de l'ensemble de connecteur de traversée (24).

2. DMA selon la revendication 1, dans lequel :
a) l'ensemble d'antenne de communication/de bobine de charge par induction (52) comprend au moins une spire de fil conducteur ou de bande ayant une longueur qui comprend des bords latéraux droit et gauche de bobine s'étendant de manière longitudinale (54, 56) rencontrant des extrémités proximale et distale de bobine opposées (58, 60) de sorte que, dans la configuration pliée au niveau de la charnière (62, 64, 66) connectée à l'un des bords latéraux droit et gauche (40, 42) de la carte de circuit imprimé (36), les bords latéraux droit et gauche de bobine (54, 56) sont alignés directement espacés au-dessus des bords latéraux droit et gauche respectifs (40, 42) et les extrémités distale et proximale de bobine opposées (58, 60) sont alignées directement espacées au-dessus des extrémités proximale et distale respectives (44, 46) de la carte de circuit imprimé ;
b) les extrémités côté dispositif des broches de borne connectées électriquement à la pluralité de contacts électriques proximaux (48A-D) au niveau de l'extrémité proximale (44) de la carte de circuit imprimé (36) sont espacées au-dessous de l'extrémité proximale (58) de l'ensemble d'antenne de communication/de bobine de charge par induction (52), adjacente à la bague métallique proximale (28) du logement en céramique (26) connectée à la férule (44) de l'ensemble de connecteur de traversée (24) ; et
c) les bornes de polarité opposée (22A-B) de la source de puissance (22) connectées aux au moins deux contacts électriques distaux (50A-B) au niveau de l'extrémité distale (46) de la carte de circuit imprimé (36) sont espacées au-dessous de l'extrémité distale (60) de l'ensemble d'antenne de communication/de bobine de charge par induction (52), adjacente à la bague métallique distale (30) du logement en céramique (26) connectée au boîtier de source de puissance.

3. DMA selon la revendication 1 ou la revendication 2, dans lequel la charnière (62, 64, 66) connectée à l'un des bords latéraux droit et gauche (40, 42) de la carte de circuit imprimé (36) est une extension flexible de la carte de circuit imprimé (36) de l'ensemble de PCB (34).

4. DMA selon l'une quelconque des revendications précédentes, dans lequel la carte de circuit imprimé (36) a une première épaisseur adjacente à l'ensemble d'antenne de communication/de bobine de charge par induction (52), et dans lequel la charnière (62, 64, 66) connectée à l'un des bords latéraux droit et gauche (40, 42) de la carte de circuit imprimé (36) a une deuxième épaisseur qui est inférieure à la première épaisseur.

5. DMA selon l'une quelconque des revendications précédentes, dans lequel la charnière (62, 64, 66) comprend au moins deux extensions flexibles espacées l'une de l'autre (62, 64, 66) de l'un des bords latéraux droit et gauche (40, 42) de la carte de circuit imprimée (36).

6. DMA selon l'une quelconque des revendications précédentes, dans lequel l'ensemble de connecteur de traversée (24) peut être connecté à un fil (74), et de préférence dans lequel une extrémité proximale du fil (74) est connectée à un dispositif de réduction de tension (76) et le dispositif de réduction de tension (76) peut être connecté à l'ensemble de connecteur de traversée (24).

7. DMA selon l'une quelconque des revendications précédentes, dans lequel la source de puissance (22) est rechargeable, et de préférence dans lequel l'ensemble d'antenne de communication/de bobine de charge par induction (52) comprend une bobine de charge connectée à un circuit de charge sur la carte de circuit imprimé (36), et dans lequel le circuit de charge est configuré pour convertir une énergie RF ou d'induction reçue de la bobine de charge en une tension de courant continu pour charger la source de puissance (22) pour alimenter en puissance l'ensemble de PCB (34).

8. Dispositif médical actif (DMA) (12), comprenant :
a) un logement en céramique (26), comprenant :
i) une paroi latérale annulaire définissant un logement ouvert s'étendant d'un rebord de logement proximal annulaire (26A) au niveau d'une extrémité ouverte proximale du logement (26) à un rebord de logement distal annulaire (26B) au niveau d'une extrémité ouverte distale du logement (26) ;
ii) une bague métallique proximale (28) brasée sur le rebord de logement proximal (26A) ; et
iii) une bague métallique distale (30) brasée sur le rebord de logement distal (26B) ;
b) un ensemble de carte de circuit imprimé (PCB) (34) logé à l'intérieur du logement en céramique (26) et comprenant une carte de circuit imprimé (36) supportant au moins un composant électronique (38), la carte de circuit imprimé (36) comprenant des bords latéraux droit et gauche s'étendant de manière longitudinale (40, 42) rencontrant des extrémités opposées proximale et distale (44, 46), dans lequel l'extrémité proximale (44) de la carte de circuit imprimé (36) supporte une pluralité de contacts électriques proximaux (48A-D), et l'extrémité distale (46) de la carte de circuit imprimé (36) supporte au moins deux contacts électriques distaux (50A-B) ;
c) un ensemble d'antenne de communication/de bobine de charge par induction (52) connecté à l'une des extrémités proximale et distale (44, 46) de la carte de circuit imprimé (36) par une charnière (62, 64, 66) et étant connecté électriquement à l'au moins un composant électronique (38) de l'ensemble de PCB (34), dans lequel, dans une configuration pliée au niveau de la charnière (62, 64, 66), l'ensemble d'antenne de communication/de bobine de charge par induction (52) est logé à l'intérieur du logement en céramique (26), espacé au-dessus de l'ensemble de PCB (34) ;
d) une source de puissance électrique (22) comprenant un boîtier métallique, dans lequel la source de puissance électrique (22) a des bornes de polarité opposée (22A-B) qui sont connectées aux au moins deux contacts électriques distaux (50A-B) au niveau de l'extrémité distale (46) de la carte de circuit imprimé (36), et dans lequel la bague métallique distale (30) au niveau de l'extrémité ouverte distale du logement en céramique (26) est soudée adjacente au boîtier de source de puissance adjacent aux contacts électriques distaux (50A-B) de la carte de circuit imprimé (26) connectés aux bornes de polarité opposée (22A-B) de la source de puissance électrique (22) ; et
e) un ensemble de connecteur de traversée (24) comprenant un isolateur non conducteur électriquement brasé sur une férule (44) dans une ouverture de férule, dans lequel l'isolateur supporte une pluralité de broches de borne scellées hermétiquement comprenant des extrémités côté dispositif de broche de borne s'étendant vers l'extérieur au-delà d'un côté dispositif d'isolateur et des extrémités côté fluide corporel de broche de borne s'étendant vers l'extérieur au-delà d'un côté fluide de corps d'isolateur, et dans lequel les extrémités côté dispositif des broches de borne sont connectées électriquement à la pluralité de contacts électriques proximaux (48A-D) au niveau de l'extrémité proximale (44) de la carte de circuit imprimé (36) adjacente au rebord de logement proximal (26A) du logement en céramique (26) brasé sur la bague proximale (28) connectée à la férule (44) de l'ensemble de connecteur de traversée (24).

9. DMA selon la revendication 8, dans lequel :
a) l'ensemble d'antenne de communication/de bobine de charge par induction (52) comprend au moins une spire de fil conducteur ou de bande ayant une longueur qui comprend des bords latéraux droit et gauche de bobine s'étendant de manière longitudinale (54, 56) rencontrant des extrémités proximale et distale de bobine opposées (58, 60) de sorte que, dans la configuration pliée au niveau de la charnière (62, 64, 66) connectée à l'une des extrémités proximale et distale (44, 46) de la carte de circuit imprimé (36), les bords latéraux droit et gauche de bobine (54, 56) sont alignés directement espacés au-dessus des bords latéraux droit et gauche respectifs (40, 42) et les extrémités distale et proximale de bobine opposées (58, 60) sont alignées directement espacées au-dessus des extrémités proximale et distale respectives (44, 46) de la carte de circuit imprimé (36) ;
b) les extrémités côté dispositif des broches de borne connectées électriquement à la pluralité de contacts électriques proximaux (48A-D) au niveau de l'extrémité proximale (44) de la carte de circuit imprimé (36) sont espacées au-dessous de l'extrémité proximale (58) de l'ensemble d'antenne de communication/de bobine de charge par induction (52), adjacente à la bague métallique proximale (28) du logement en céramique (26) connectée à la férule (44) de l'ensemble de connecteur de traversée (24) ; et
c) les bornes de polarité opposée (22A-B) de la source de puissance (22) connectées aux au moins deux contacts électriques distaux (50A-B) au niveau de l'extrémité distale (46) de la carte de circuit imprimé (36) sont espacées au-dessous de l'extrémité distale (60) de l'ensemble d'antenne de communication/de bobine de charge par induction (52), adjacente à la bague métallique distale (30) du logement en céramique (26) connectée au boîtier de source de puissance.

10. DMA selon la revendication 8 ou la revendication 9, dans lequel la charnière (62, 64, 66) connectant l'une des extrémités proximale et distale (44, 46) de la carte de circuit imprimé (36) à l'ensemble d'antenne de communication/de bobine de charge par induction (52) est une charnière vivante.

11. DMA selon l'une quelconque des revendications 8 à 10, dans lequel la charnière (62, 64, 66) connectée à l'une des extrémités proximale et distale (44, 46) de la carte de circuit imprimé (36) est une extension flexible de la carte de circuit imprimé (36) de l'ensemble de PCB (34), ou dans lequel la charnière (62, 64, 66) comprend au moins deux extensions flexibles espacées l'une de l'autre (62, 64, 66) de l'une des extrémités proximale et distale (44, 46) de la carte de circuit imprimé (36).

12. DMA selon l'une quelconque des revendications 8 à 11, dans lequel la carte de circuit imprimé (36) a une première épaisseur adjacente à l'ensemble d'antenne de communication/de bobine de charge par induction (52), et la charnière (62, 64, 66) connectée aux extrémités proximale et distale (44, 46) de la carte de circuit imprimé (36) a une deuxième épaisseur qui est inférieure à la première épaisseur.

13. DMA selon l'une quelconque des revendications 8 à 12, dans lequel l'ensemble de connecteur de traversée (24) peut être connecté à un fil (74), et de préférence dans lequel une extrémité proximale du fil (74) est connectée à un dispositif de réduction de tension (76) et le dispositif de réduction de tension (76) peut être connecté à l'ensemble de connecteur de traversée (24).

14. DMA selon l'une quelconque des revendications 8 à 13, dans lequel la source de puissance (22) est rechargeable, et de préférence dans lequel l'ensemble d'antenne de communication/de bobine de charge par induction (52) comprend une bobine de charge connectée à un circuit de charge sur la carte de circuit imprimé (36), et dans lequel le circuit de charge est configuré pour convertir une énergie RF ou d'induction reçue de la bobine de charge en une tension de courant continu pour charger la source de puissance (22) pour alimenter en puissance l'ensemble de PCB (34).

15. DMA selon l'une quelconque des revendications 8 à 14, dans lequel l'ensemble d'antenne de communication/de bobine de charge par induction (52) a une forme sélectionnée parmi une forme rectangulaire, une forme ovale, une forme circulaire, une forme à côtés multiples qui n'est ni rectangulaire ni circulaire mais qui a à la fois des côtés droits et des côtés incurvés, et dans lequel l'ensemble d'antenne de communication/de bobine de charge par induction (52) a ou n'a pas une longueur et une largeur qui s'alignent sensiblement avec une longueur et une largeur de l'ensemble de PCB (34).
